# EUROPÄISCHE PATENTANMELDUNG

(11) **EP 4 354 168 A1**
(43) Veröffentlichungstag der Anmeldung: **17.04.2024**
(21) Anmeldenummer: 22201229.6
(22) Anmeldetag: 13.10.2022
(51) Int. Cl.: G01R 33/54, G01R 33/48, G01R 33/56

(54) **VERFAHREN ZUM NACHWEIS EINES VORGEGEBENEN NACHZUWEISENDEN STOFFES IN EINEM UNTERSUCHUNGSOBJEKT, STEUEREINRICHTUNG, MAGNETRESONANZVORRICHTUNG, COMPUTERPROGRAMM UND ELEKTRONISCH LESBARER DATENTRÄGER**

(71) Anmelder: Siemens Healthineers AG, 91301 Forchheim (DE)
(72) Erfinder: Gall, Peter, 91080 Uttenreuth (DE); Körzdörfer, Gregor, 91054 Erlangen (DE); Leidecker, Christianne, 90419 Nürnberg (DE); Liu, Wei, Shenzhen, 518055 (CN); Maier, Florian, 91054 Buckenhof (DE); Meyer, Heiko, 91080 Uttenreuth (DE); Polak, Daniel, 91052 Erlangen (DE); Splitthoff, Daniel Nicolas, 91080 Uttenreuth (DE); Vahle, Thomas, 90409 Nürnberg (DE)
(74) Vertreter: Siemens Healthineers Patent Attorneys

(57) **Zusammenfassung**

Die Erfindung betrifft ein Verfahren zum Nachweis eines vorgegebenen Stoffes in einem Untersuchungsobjekt (100) durch eine Magnetresonanzvorrichtung (1), wobei durch eine Steuereinrichtung (20) eine Magnetresonanzsequenz (MRT) umfassend zumindest eine Untersequenz (MRT1, MRT2) zum Nachweis zumindest eines nachzuweisenden Stoffes in dem Untersuchungsobjekt (100) in Abhängigkeit von dem zumindest einen nachzuweisenden Stoff ermittelt wird, zumindest ein Messzeitpunkt (T1, T2, T3) zum Erfassen eines jeweiligen MRT-Signals (A1, A2, A3) zum Nachweisen des zumindest einen nachzuweisenden Stoffes in dem Untersuchungsobjekt (100) in Abhängigkeit von dem zumindest einen nachzuweisenden Stoff ermittelt wird. Es ist vorgesehen, dass das zumindest eine MRT-Signal (A1, A2, A3) auf eine Erfüllung einer vorbestimmten Nachweisbedingung ausgewertet wird, und bei einer Erfüllung der vorbestimmten Nachweisbedingung durch das zumindest eine MRT-Signal (A1, A2, A3) eine Anwesenheit des zumindest einen nachzuweisenden Stoffes festgestellt wird.

## Beschreibung

Die Erfindung betrifft ein Verfahren zum Nachweis eines vorgegebenen nachzuweisenden Stoffes in einem Untersuchungsobjekt durch eine Magnetresonanzvorrichtung, eine Steuereinrichtung, eine Magnetresonanzvorrichtung, ein Computerprogramm sowie ein elektronisch lesbarer Datenträger.

Vorrichtungen zur Magnetresonanztomographie, MRT, sind bildgebende Vorrichtungen, die ein starkes äußeres Magnetfeld nutzen, um die Kernspins eines zu untersuchenden Objekts auszurichten und sie durch Anlegen eines HF-Anregungspulses zur Präzession um die entsprechende Ausrichtung anzuregen. Die Präzession beziehungsweise der Übergang der Spins von diesem angeregten Zustand in einen Zustand mit geringerer Energie erzeugt als Antwort ein elektromagnetisches Wechselfeld, das über Empfangsantennen als MR-MRT-Signal detektiert werden kann.

Mit Hilfe von magnetischen Gradientenfeldern kann den Signalen eine Positionskodierung aufgeprägt werden, die es anschließend erlaubt, das erhaltene MRT-Signal einem Volumenelement des Untersuchungsobjekts zuzuordnen. Das empfangene MRT-Signal kann dann ausgewertet werden, um beispielsweise eine Bilddarstellung des Untersuchungsobjekts bereitzustellen.

Zur Anregung von Untersuchungsobjekten ist es vorgesehen, dass die HF-Anregungspulse und die Gradientenfelder in einer Magnetfeldresonanzsequenz bereitgestellt werden. Die Magnetfeldresonanzsequenz kann durch Sequenzparameter, wie zum Beispiel einer Repetitionszeit TR, einer Echozeit TE, einer Inversionszeit TI und einem Flipwinkel α gekennzeichnet sein.

Diese beeinflussen die Signalintensität des empfangenen MRT-Signals aus einem Volumenelement. Diese Signalintensität hängt aber auch von den in dem Volumenelement enthaltenen Stoffen ab, wobei sich die Gesamtsignalintensität aus den Signalintensitäten der einzelnen Stoffe zusammen. Die jeweiligen Signalintensitäten der Stoffe hängen unter anderem von den Relaxationszeiten T1 und T2 dieser Stoffe ab. Durch eine geeignete Wahl der Sequenzparameter der Magnetresonanzsequenz kann die Anregung und Signalaufnahme derart angepasst werden, dass die Signalintensitäten beispielsweise T1- oder T2-gewichtet abgebildet werden können.

Magnetresonanzuntersuchungen werden beispielsweise zur Bildgebung angewandt, um eine zweidimensionale oder dreidimensionale Abbildung des Untersuchungsobjektes zu ermöglichen. Dabei werden unterschiedliche Signalintensitäten beispielsweise mit Hilfe unterschiedlicher Grauwerte in der Abbildung abgebildet. Die lokale Signalintensität hängt unter anderem von den lokal anwesenden Stoffen ab. Anhand der lokalen Signalintensität ist es daher in einem gewissen Maß möglich, lokal anwesende Stoffe oder Gewebearten zu identifizieren.

Das Ziel der klinischen Erfassung von MRT-Aufnahmen besteht praktisch immer darin, die Physiologie oder Anatomie in diagnostische Informationen umzuwandeln. Dies bedeutet, dass basierend auf den MRT-Aufnahmen Pathologien erkannt und deren Schweregrad abzuschätzen ist. Insbesondere im Falle der beschriebenen Bildgebung ist eine Interpretation und Bewertung von Bildrekonstruktionen durch Radiologen erforderlich, um beispielsweise eine Anwesenheit von bestimmten Stoffen oder krankhaftem Gewebe feststellen zu können.

Bislang wird die Anwesenheit eines bestimmten Stoffes oder Gewebes in dem Untersuchungsobjekt in den meisten Fällen durch die Rekonstruktion von Bildern und deren Bewertung durch Radiologen überprüft. Es wurden zwar erste Ansätze für eine automatische Bildanalyse vorgeschlagen, um bestimmte Stoffe automatisch identifizieren zu können, darunter auch KI-gestützte Verfahren, jedoch werden weiterhin Bilder als Ausgangspunkt zum Nachweis bestimmter Stoffe verwendet.

Ein weiterer Ansatz sieht eine Anwendung spektroskopischer Verfahren vor, um bestimmte Stoffe oder bestimmtes Gewebe nachzuweisen und somit eine Bösartigkeit von Gewebe beurteilen zu können. Hier wird die Signalintensität von Stoffen mit einer für diese charakteristischen Resonanzfrequenz gemessen und diese beispielsweise in Form eines Resonanzfrequenz-Intensitäts-Diagramms dargestellt. Allerdings lassen sich damit nur Informationen aus einem relativ großen Volumenelement erfassen. Die Erfassung ist zudem relativ langsam.

Ferner sind auch Ansätze bekannt, welche eine Ermittlung eines anwesenden Stoffes in dem Untersuchungsobjekt beispielsweise mittels eines sogenannte Magnetresonanz-Fingerprinting-Verfahrens ermöglichen. Zur Durchführung eines Nachweises eines Stoffes mittels dieses Verfahrens ist es vorgesehen, zu mehreren Zeitpunkten Signale des Untersuchungsobjektes zu erfassen, um einen zeitlichen Verlauf einer Gesamtsignalintensität an einem Bildpunkt zu ermitteln. Dieser Signalzeitverlauf wird dann mit simulierten Signalzeitverläufen verglichen, die generiert wurden, indem bestimmte Annahmen über die Gewebeeigenschaften (zum Beispiel T1- und T2-Werte) bei der Simulation vorgegeben wurden.

Magnetresonanz-Fingerprinting-Verfahren brechen das herkömmliche Paradigma der Datenerfassung durch Anwendung regelmäßiger Magnetresonanzsequenzen von Hochfrequenz- und Gradientenimpulsen auf. Zur Durchführung des Magnetresonanz-Fingerprinting-Verfahrens werden die Hochfrequenzsequenz und die Gradientensequenz der Magnetresonanzsequenz so optimiert, dass die erfassten, lokalisierten Bilddaten nicht nur ein einzelnes Bild liefern, sondern vielmehr eine Serie von Bildern. Aus dieser Serie werden Signalzeitverläufe extrahiert, die mit einem computergenerierten Wörterbuch simulierter Signalzeitverläufe der Signalintensitäten für die entsprechende Magnetresonanzsequenz auf der Grundlage der verschiedenen Stoffparameter der Stoffe, wie den Relaxationszeiten T1, T2 und der chemischen Verschiebung abgeglichen werden können. Durch den Abgleich der erfassten Signalzeitverläufe mit bekannten Verläufen von Signalintensitäten für jeweilige Stoffparameter ist es möglich, diese Stoffparameter, wie T1 oder T2, zu identifizieren. Bei Durchführung des Magnetresonanz-Fingerprinting-Verfahrens erfolgt die Datenerfassung für gewöhnlich nicht durch Anwendung regelmäßiger Magnetresonanzsequenzen von Hochfrequenz- und Gradientenimpulsen.

Ein mögliches Magnetresonanz-Fingerprinting Verfahren ist beispielsweise in der DE 10 2019 206 827 A1 offenbart. In dem Verfahren ist es vorgesehen, Parameterwerte in Bildpunkten eines Untersuchungsvolumens eines Untersuchungsobjektes in einer MR-Anlage mittels einer Magnetresonanz-Fingerprinting-Technik zu ermitteln. In dem Verfahren wird mindestens eine Bildpunkt-Zeit-Serie des Untersuchungsobjekts mithilfe eines MRF-Aufnahmeverfahrens der Art durchgeführt, dass erfasste Bildpunkt-Zeit-Serien mit geladenen Vergleichssignalverläufen vergleichbar sind. Es erfolgt ein Signalvergleich mindestens eines Abschnitts des jeweiligen Signalverlaufs der erfassten Bildpunkt-Zeit-Serien mit einem entsprechenden Abschnitt von geladenen Vergleichssignalverläufen zur Ermittlung von Ähnlichkeitswerten der erfassten Bildpunkt-Zeit-Serie mit den jeweiligen Vergleichssignalverläufen.

Nachteilig an Magnetresonanz-Fingerprinting-Verfahren ist es, dass die dazu erforderlichen Magnetresonanzsequenzen relativ lang sind, um eine Bildpunkt-Zeit-Serie bereitstellen zu können.

Es ist somit eine Aufgabe der Erfindung, ein MRT-Verfahren bereitzustellen, welches eine kürzere Zeit zur Ermittlung einer Anwesenheit eines bestimmten Stoffes in einem Untersuchungsobjekt benötigt.

Diese Aufgabe wird gelöst durch den jeweiligen Gegenstand der unabhängigen Ansprüche. Vorteilhafte Weiterbildungen und bevorzugte Ausführungsformen sind Gegenstand der abhängigen Ansprüche.

Ein erster Aspekt der Erfindung betrifft ein Verfahren zum Nachweis eines vorgegebenen nachzuweisenden Stoffes in einem Untersuchungsobjekt durch eine Magnetresonanzvorrichtung.

In einem ersten Schritt wird durch eine Steuereinrichtung eine Anweisung zum Nachweisen zumindest eines der in dem Untersuchungsobjekt potentiell vorhandenen Stoffe empfangen. Mit anderen Worten wird der Steuereinrichtung vorgegeben, welcher potentiell vorhandene Stoff auf eine Anwesenheit in dem Untersuchungsobjekt durch die Magnetresonanzvorrichtung zu überprüfen ist.

Durch die Steuereinrichtung wird dementsprechend eine Durchführung einer Magnetresonanzuntersuchung zum Nachweis des Stoffes in dem Untersuchungsobjekt vorbereitet.

Dazu wird in einem Schritt des Verfahrens durch die Steuereinrichtung eine Magnetresonanzsequenz, welche zumindest eine Untersequenz umfasst, zum Nachweis des zumindest einen nachzuweisenden Stoffes in dem Untersuchungsobjekt ermittelt. Die zumindest eine Untersequenz kann eine regelmäßige oder unregelmäßige Folge von Echo und/oder Gradientensequenzen umfassen. Dabei können beispielsweise Flipwinkel und/oder Repetitionszeiten variiert sein. Die zumindest eine Untersequenz kann ein zeitlicher Abschnitt der Magnetresonanzsequenz sein. Mit anderen Worten wird durch die Steuereinrichtung die Magnetresonanzsequenz generiert oder abgerufen, welche durch die Magnetresonanzvorrichtung durchzuführen ist, um den zumindest einen nachzuweisenden Stoff in dem Untersuchungsobjekt nachzuweisen. Mit anderen Worten erfolgt die Generierung der Magnetresonanzsequenz durch die Steuereinrichtung in Abhängigkeit von dem zumindest einen in dem Untersuchungsobjekt nachzuweisenden Stoff. Entsprechend dem nachzuweisenden Stoff wird die Magnetresonanzsequenz ermittelt, welche zum Erfassen des nachzuweisenden Stoffes in dem Untersuchungsobjekt geeignet ist. Es kann beispielsweise vorgesehen sein, dass ein simulierter oder gespeicherter Verlauf einer Signalintensität für den nachzuweisenden Stoff bei der Ermittlung der Magnetresonanzsequenz berücksichtigt wird. Die Magnetresonanzsequenz kann Anregungszeiten und Messzeiten umfassen.

Es ist vorgesehen, dass durch die Steuereinrichtung zumindest ein Messzeitpunkt in der Magnetresonanzsequenz zum Erfassen eines jeweiligen MRT-Signals zum Nachweis des zumindest einen nachzuweisenden Stoffes in dem Untersuchungsobjekt ermittelt wird. Der zumindest eine Messzeitpunkt kann eine Echozeit der Magnetresonanzsequenz sein, zu der das jeweilige MRT-Signal zu erfassen ist. Der zumindest eine Messzeitpunkt wird durch die Steuereinrichtung in Abhängigkeit von dem zumindest einen nachzuweisenden Stoff ermittelt. Mit anderen Worten erfolgt durch die Steuereinrichtung die Ermittlung des zumindest einen Messzeitpunktes, an welchem durch die Magnetresonanzvorrichtung das jeweilige MRT-Signal zu erfassen ist, um die Anwesenheit des zumindest einen nachzuweisenden Stoffes in dem Untersuchungsobjekt ermöglichen zu können. Das MRT-Signal kann eine Gesamtsignalintensität aufweisen, welche sich aus Signalintensitäten von Stoffen des Untersuchungsobjektes zusammensetzen kann. Der zumindest eine Messzeitpunkt kann beispielsweise ein Echozeitpunkt der Magnetresonanzsequenz, oder ein ausgewählter Zeitpunkt der Magnetresonanzsequenz sein, bei dem die MRT-Signale besonders gut erfasst werden können. Der zumindest eine Messzeitpunkt wird dabei ebenfalls unter Berücksichtigung des zumindest einen nachzuweisenden Stoffes ermittelt.

Nach der Ermittlung der Magnetresonanzsequenz und des zumindest einen Messzeitpunktes wird die Magnetresonanzvorrichtung durch die Steuereinrichtung angesteuert, um die ermittelte Magnetresonanzsequenz durch die Magnetresonanzvorrichtung bereitzustellen.

In einem folgenden Schritt wird die Magnetresonanzvorrichtung zur Erfassung des zumindest einen MRT-Signals des Untersuchungsobjekts zum Nachweisen nachzuweisender Stoffe zu dem zumindest einen Messzeitpunkt in der Magnetresonanzsequenz angesteuert. Bei dem zumindest einen Messzeitpunkt kann es sich beispielsweise um den Echozeitpunkt handeln, oder einen ausgewählten Zeitpunkt in der Magnetresonanzsequenz, bei dem die MRT-Signale besonders gut erfasst werden können.

In einem folgenden Schritt wird das zumindest eine MRT-Signal durch die Steuereinrichtung empfangen und eine Anwesenheit des zumindest einen nachzuweisenden Stoffes bei einer Erfüllung einer vorbestimmten Nachweisbedingung durch das zumindest eine MRT-Signal festgestellt. Mit anderen Worten empfängt die Steuereinrichtung das durch die Magnetresonanzvorrichtung erfasst MRT-Signal. Durch die Steuereinrichtung erfolgt eine Auswertung des empfangenen MRT-Signals, um zu überprüfen, ob der nachzuweisende Stoff in dem Untersuchungsobjekt vorhanden ist. Die Anwesenheit des nachzuweisenden Stoffes in dem Untersuchungsobjekt wird durch die Steuereinrichtung festgestellt, wenn die vorbestimmte Nachweisbedingung durch das zumindest eine MRT-Signal erfüllt ist. Die vorbestimmte Nachweisbedingung kann beispielsweise ein Überschreiten eines vorbestimmten Schwellenwertes durch die vorbestimmte Gesamtsignalintensität des zumindest einen MRT-Signals vorgeben.

Bei der Erfüllung der vorbestimmten Bedingung kann durch die Steuereinrichtung ein vorbestimmter Verfahrensschritt eingeleitet werden. Es kann beispielsweise eine Ausgabe eines Nachweissignals erfolgen, welche den oder die nachgewiesenen Stoffe anzeigen kann. Wird die vorbestimmte Bedingung nicht erfüllt, kann eine Ausgabe eines anderen Nachweissignals vorgesehen sein, das anzeigt, dass keiner der nachzuweisenden Stoffe nachgewiesen werden konnte. Es kann auch eine Einleitung weiterer Schritte, wie einer Durchführung oder einer Anpassung einer weiteren Sequenz bei einem Nachweis des zumindest einen nachzuweisenden Stoffes vorgesehen sein.

Durch die Erfindung ergibt sich der Vorteil, dass zum Nachweis des zumindest einen nachzuweisenden Stoffes eine dafür ermittelte Magnetresonanzsequenz und ein dafür ermittelter Messzeitpunkt verwendet werden, welche auf den jeweiligen nachzuweisenden Stoff optimiert sind.

Dadurch ist ein effizienterer Nachweis einer Anwesenheit des zumindest einen nachzuweisenden Stoffes im Untersuchungsobjekt möglich, als es bei stoffunabhängigen Magnetresonanzsequenzen der Fall ist. Dadurch ist eine schnellere Erfassung einer Anwesenheit des Stoffes möglich, als es beispielsweise bei derzeit üblichen Fingerprinting-Verfahren der Fall ist, weil bei Fingerprinting-Verfahren immer eine Zeitserie von mehreren MRT-Signalen erforderlich ist. Der Kerngedanke der Erfindung besteht darin, dass die Messung nicht notwendigerweise räumlich aufgelöst erfolgen muss, wodurch Messzeit gegenüber Fingerprinting-Verfahren eingespart werden kann. Die Messung wird derartig parametrisiert, dass diese sehr sensitiv auf den bestimmten nachzuweisenden Stoff ist, und damit keine weiteren Messungen mehr durchgeführt werden müssen, wenn der nachzuweisende Stoff nicht detektiert wird.

Die Erfindung umfasst auch Weiterbildungen, durch die sich weitere Vorteile ergeben.

Eine Weiterbildung der Erfindung sieht vor, dass durch die Steuereinrichtung eine Information über zumindest einen weiteren Stoff, welcher potentiell in dem Untersuchungsobjekt vorhanden ist, empfangen wird. Mit anderen Worten empfängt die Steuereinrichtung Informationen darüber welcher zumindest eine weitere Stoff sich zusätzlich zu dem zumindest einen nachzuweisenden Stoff in dem Untersuchungsobjekt befindet oder befinden könnte.

Durch die Steuereinrichtung wird die Magnetresonanzsequenz, welche die zumindest eine Untersequenz umfasst, zum Nachweis des zumindest einen nachzuweisenden Stoffes in dem Untersuchungsobjekt in Abhängigkeit von dem zumindest einen potentiell vorhandenen weiteren Stoffermittelt. Mit anderen Worten wird die Magnetresonanzsequenz durch die Steuereinrichtung unter Berücksichtigung des zumindest einen potentiell vorhandenen weiteren Stoff ermittelt. Mit anderen Worten wird bei der Generierung der Magnetresonanzsequenz durch die Steuereinrichtung berücksichtigt, welcher potentiell vorhandene Stoffe in dem Untersuchungsobjekt vorhanden ist, um entsprechend dem potentiell vorhandenen Stoff die Magnetresonanzsequenz zu ermitteln, welche zum Erfassen des nachzuweisenden Stoffes unter Anwesenheit des zumindest einen weiteren Stoffes des Untersuchungsobjektes geeignet ist. Es kann beispielsweise berücksichtigt werden, wie neben dem nachzuweisenden Stoff der zumindest eine weitere Stoff durch die Magnetresonanzsequenz angeregt wird. Es kann beispielsweise vorgesehen sein, dass ein Verlauf einer Signalintensität für den weiteren Stoff in Abhängigkeit der Magnetresonanzsequenz berücksichtigt wird.

Es ist vorgesehen, dass durch die Steuereinrichtung zumindest ein Messzeitpunkt zum Erfassen eines jeweiligen MRT-Signals zum Nachweis des zumindest einen nachzuweisenden Stoffes in dem Untersuchungsobjekt ermittelt wird. Der zumindest eine Messzeitpunkt wird durch die Steuereinrichtung in Abhängigkeit von dem zumindest einen nachzuweisenden Stoff und dem potentiell vorhandenen weiteren Stoff ermittelt. Mit anderen Worten erfolgt durch die Steuereinrichtung die Ermittlung des zumindest einen Messzeitpunktes, an welchem durch die Magnetresonanzvorrichtung das jeweilige MRT-Signal zu erfassen ist, um die Anwesenheit des zumindest einen nachzuweisenden Stoffes in dem Untersuchungsobjekt ermöglichen zu können. Das MRT-Signal kann eine Gesamtsignalintensität aufweisen, welche sich aus den Signalintensitäten des nachzuweisenden Stoffes und des zumindest einen weiteren Stoffes zusammensetzen kann. Der zumindest eine Messzeitpunkt kann beispielsweise eine Echozeit der Magnetresonanzsequenz sein. Der zumindest eine Messzeitpunkt wird dabei ebenfalls unter Berücksichtigung des nachzuweisenden Stoffes und des zumindest einen weiteren Stoffes ermittelt.

Eine Weiterbildung der Erfindung sieht vor, dass die Untersequenz zum Nachweis des zumindest einen nachzuweisenden Stoffes in dem Untersuchungsobjekt nach einem vorbestimmten Sequenzgenerierungsalgorithmus in Abhängigkeit von Stoffparametern des zumindest einen nachzuweisenden Stoffes und/oder des zumindest einen potentiell vorhandenen weiteren Stoffes ermittelt wird. Es ist vorgesehen, dass der vorbestimmte Sequenzgenerierungsalgorithmus dazu eingerichtet ist, die Untersequenz gemäß einem vorgegebenen Optimierungskriterium zu generieren. Das vorgegebene Optimierungskriterium kann vorgeben, dass die Untersequenz durch den Sequenzgenerierungsalgorithmus dahingehend zu optimieren ist, dass ein Verlauf einer Signalintensität des MRT-Signals für den zumindest einen nachzuweisenden Stoff ein vorbestimmtes Unterscheidungskriterium in Bezug auf einen jeweiligen Verlauf einer Signalintensität des MRT-Signals für den zumindest einen potentiell vorhandenen weiteren Stoff erfüllt. Mit anderen Worten ist es vorgesehen, dass durch die Steuereinrichtung nach dem vorbestimmten Sequenzgenerierungsalgorithmus die Untersequenz zum Nachweis des zumindest einen nachzuweisenden Stoffes ermittelt wird. Der Sequenzgenerierungsalgorithmus kann beispielsweise eine mathematische Funktion, ein Modell oder eine Simulation umfassen, welche dazu vorgesehen sind, die Untersequenz in Abhängigkeit von den Stoffparametern der potentiell vorhandenen Stoffe zu ermitteln. Die Stoffparameter der potentiell vorhandenen Stoffe können beispielsweise eine T1, eine T2 oder eine T2* Relaxationszeit oder auch Parameter wie ADC oder Offresonanz umfassen. Mit anderen Worten handelt es sich bei den Stoffparametern um Eingangsgrößen des vorbestimmten Sequenzgenerierungsalgorithmus. Der vorbestimmte Sequenzgenerierungsalgorithmus kann dazu vorgesehen sein, die Untersequenz in Abhängigkeit von den Stoffparametern derart zu generieren, dass ein Verlauf der Signalintensität des MRT-Signals für den zumindest einen nachzuweisenden Stoff ein vorbestimmtes Unterscheidungskriterium erfüllt, um einen Nachweis des Stoffes in dem MRT-Signal ermöglichen zu können. Das Unterscheidungskriterium kann beispielsweise Vorgaben in Bezug auf die Verläufe der Signalintensitäten und oder einzelner Zeitpunkte der Verläufe umfassen. Bei der Funktion des Sequenzgenerierungsalgorithmus kann es sich beispielsweise um eine Maximierungsfunktion handeln. Der Sequenzgenerierungsalgorithmus kann beispielsweise dazu ausgelegt sein, eine Untersequenz für eine möglichst große Signalintensität und/oder einen möglichst großen Signalintensitätsanteil der Signalintensität des zumindest einen nachzuweisenden Stoffes an einer Gesamtsignalintensität bereitzustellen. Der Sequenzgenerierungsalgorithmus kann auch dazu ausgelegt sein, die Untersequenz zu ermitteln, welche Signalverläufe für die Stoffe bewirkt, die sich möglichst voneinander unterscheiden lassen.

Eine Weiterbildung der Erfindung sieht vor, dass das vorbestimmte Optimierungskriterium eine Maximierung einer Signalintensität des MRT-Signals für den zumindest einen nachzuweisenden Stoff an zumindest einem Zeitpunkt der Magnetresonanzsequenz vorgibt. Mit anderen Worten ist es vorgesehen, dass das vorbestimmte Optimierungskriterium eine Bereitstellung einer Untersequenz vorgibt, welche eine maximale Signalintensität des MRT-Signals des nachzuweisenden Stoffes an zumindest einem Zeitpunkt der Magnetresonanzsequenz bewirkt. Mit anderen Worten schreibt das Optimierungskriterium vor, dass der Verlauf der Signalintensität des MRT-Signals des nachzuweisenden Stoffes an zumindest einem Zeitpunkt der Magnetresonanzsequenz ein möglichst großes Maximum aufweist.

Eine Weiterbildung der Erfindung sieht vor, dass das vorbestimmte Optimierungskriterium eine Minimierung einer Signalintensität des MRT-Signals für den zumindest einen potentiell vorhandenen weiteren Stoff an zumindest einem Zeitpunkt der Magnetresonanzsequenz vorgibt. Mit anderen Worten ist es vorgesehen, dass das vorbestimmte Optimierungskriterium eine Bereitstellung einer Untersequenz vorgibt, welche eine minimale Signalintensität des MRT-Signals des potentiell vorhandenen weiteren Stoffes an zumindest einem Zeitpunkt der Magnetresonanzsequenz bewirkt.

Eine Weiterbildung der Erfindung sieht vor, dass das vorbestimmte Optimierungskriterium eine Maximierung eines Signalanteils der Signalintensität an einer Gesamtsignalintensität des MRT-Signals für den zumindest einen nachzuweisenden Stoff an zumindest einem Zeitpunkt der Magnetresonanzsequenz vorgibt. Mit anderen Worten gibt das Optimierungskriterium vor, dass ein Signalanteil der Signalintensität des nachzuweisenden Stoffes an zumindest einem Zeitpunkt der Magnetresonanzsequenz ein Maximum aufweist. Mit anderen Worten ist die Magnetresonanzsequenz dahingehend zu optimieren, dass die Verläufe der jeweiligen Signalintensitäten der jeweiligen Stoffe derart verlaufen, dass die Signalintensität des MRT-Signals des zumindest einen nachzuweisenden Stoffes an zumindest einem Zeitpunkt der Magnetresonanzsequenz einen maximalen Anteil an einer Gesamtintensität aufweist. Bevorzugt ist angestrebt, dass zu zumindest einem Zeitpunkt der Magnetresonanzsequenz die Gesamtsignalintensität mit der Signalintensität des zumindest einen nachzuweisenden Stoffes übereinstimmt. Mit anderen Worten ist es bevorzugt vorgesehen, dass zu dem zumindest einen Zeitpunkt der Magnetresonanzsequenz die Signalintensitäten der weiteren Stoffe unterdrückt sind.

Eine Weiterbildung der Erfindung sieht vor, dass das vorbestimmte Optimierungskriterium eine Minimierung eines Signalanteils der Signalintensität an einer Gesamtsignalintensität des MRT-Signals für den zumindest einen potentiell vorhandenen weiteren Stoff an zumindest einem Zeitpunkt der Magnetresonanzsequenz vorgibt. Mit anderen Worten gibt das Optimierungskriterium vor, dass ein Signalanteil der Signalintensität des potentiell vorhandenen weiteren Stoffes an zumindest einem Zeitpunkt der Magnetresonanzsequenz ein Minimum aufweist. Mit anderen Worten ist die Magnetresonanzsequenz dahingehend zu optimieren, dass die Verläufe der jeweiligen Signalintensitäten der jeweiligen Stoffe derart verlaufen, dass die Signalintensität des MRT-Signals des zumindest einen potentiell vorhandenen weiteren Stoffes an zumindest einem Zeitpunkt der Magnetresonanzsequenz einen minimalen Anteil an einer Gesamtintensität aufweist

Eine Weiterbildung der Erfindung sieht vor, dass durch die Steuereinrichtung für den zumindest einen nachzuweisenden Stoff zumindest zwei mögliche Untersequenzen zum Nachweis des jeweiligen Stoffes ermittelt werden. Mit anderen Worten werden für die jeweiligen nachzuweisenden Stoffe jeweils zumindest zwei mögliche Untersequenzen durch die Steuereinrichtung generiert und/oder aus einer Datenbank abgerufen. Die möglichen Untersequenzen können beispielsweise nach jeweiligen verschiedenen Algorithmen generiert worden sein oder aus unterschiedlichen Bibliotheken abgerufen sein und sich somit voneinander Unterscheiden. Die Untersequenzen können insbesondere für die vorhandenen Stoffe vorgesehen sein und/oder zum Nachweis des zumindest einen nachzuweisenden Stoffes. Dadurch können zumindest zwei Untersequenzen bereitgestellt sein, welche zum Nachweis des zumindest einen nachzuweisenden Stoffes geeignet sind. Nun kann es vorgesehen sein, diejenige der möglichen Untersequenzen zu identifizieren und auszuwählen, welche für die Messung geeigneter ist. In einem weiteren Schritt ist es vorgesehen, dass durch die Steuereinrichtung für die zumindest zwei möglichen Untersequenzen jeweilige Verläufe jeweiliger Signalintensitäten des MRT-Signals für die jeweiligen Stoffe über eine Zeit der möglichen Untersequenzen ermittelt werden. Mit anderen Worten wird durch die Steuereinrichtung ermittelt, welche Verläufe jeweilige Signalintensitäten aufweisen, welche auf die jeweiligen Stoffe zurückzuführen sind. Die Verläufe können beispielsweise berechnet, simuliert oder abgerufen werden. In einem weiteren Schritt kann durch die Steuereinrichtung eine der zumindest zwei möglichen Untersequenzen in Abhängigkeit von zumindest einem der Signalverläufe nach einem vorbestimmten Auswahlkriterium als Untersequenz zum Nachweis des zumindest einen nachzuweisenden Stoffes ausgewählt werden. Mit anderen Worten ist es vorgesehen, dass eine der zumindest zwei möglichen Untersequenzen zum Nachweis des jeweiligen Stoffes in dem Untersuchungsobjekt durch die Steuereinrichtung ausgewählt wird. Die Auswahl erfolgt nach einem vorbestimmten Auswahlkriterium. Das Auswahlkriterium kann vorgeben, welche der möglichen Untersequenzen gewählt wird, und auf die jeweilige Messung oder den den jeweiligen nachzuweisenden Stoff optimiert sein. Das vorbestimmte Auswahlkriterium kann beispielsweise auf den Signalverlauf der Signalintensität des MRT-Signals für den nachzuweisenden Stoff oder die Signalverläufe der Signalintensitäten sämtlicher Stoffe bezogen sein.

Eine Weiterbildung der Erfindung sieht vor, dass durch die Steuereinrichtung für die jeweiligen möglichen Untersequenzen ein jeweiliger Maximalwert eines Signalintensitätsanteils der Signalintensität des MRT-Signals für den zumindest einen nachzuweisenden Stoff an einer Gesamtsignalintensität des MRT-Signals ermittelt wird, wobei die Gesamtintensität des MRT-Signals sich aus den Signalintensitäten der jeweiligen in dem Volumenelement enthaltenen und zu dem Signal beitragenden Stoffe zusammensetzt. Das Auswahlkriterium gibt vor, dass diejenige der möglichen Untersequenzen als Untersequenz ausgewählt wird, welche den größten Maximalwert des Signalintensitätsanteils des nachzuweisenden Stoffes aufweist. Mit anderen Worten wird die mögliche Untersequenz als Untersequenz zum Nachweis des jeweiligen nachzuweisenden Stoffes ausgewählt, welche einen größten Maximalwert des Signalintensitätsanteils des nachzuweisenden Stoffes hervorruft. Mit anderen Worten werden für die jeweiligen möglichen Untersequenzen die jeweiligen Signalintensitätsanteile ermittelt, welche die einzelnen Stoffe an zumindest einem Zeitpunkt aufweisen. Es kann vorgesehen sein, dass der jeweilige Signalintensitätsanteil über eine gesamte Zeit der jeweiligen Untersequenzen, an mehreren zufälligen oder bestimmten Zeitpunkten der jeweiligen Untersequenzen oder einem einzigen Zeitpunkt ermittelt wird. Die Gesamtintensität des MRT-Signals kann sich aus den Signalintensitäten der potentiell vorhandenen Stoffe zusammensetzen. Dabei kann für jede der möglichen Untersequenzen der jeweilige Maximalwert ermittelt werden, welcher den Signalintensitätsanteil des nachzuweisenden Stoffes in der jeweiligen möglichen Untersequenz aufweist. Das Auswahlkriterium kann vorsehen, dass die Untersequenz ausgewählt wird, deren Maximalwert des Signalintensitätsanteils des nachzuweisenden Stoffes am größten ist. Dadurch ergibt sich der Vorteil, dass die Untersequenz ausgewählt wird, welche einen maximalen Intensitätsanteil des nachzuweisenden Stoffes aufweist.

Eine Weiterbildung der Erfindung sieht vor, dass für die Magnetresonanzsequenz jeweilige Verläufe jeweiliger Signalintensitäten des MRT-Signals für die jeweiligen Stoffe über eine Zeit der ermittelten Magnetresonanzsequenz ermittelt werden. In einem weiteren Schritt wird zumindest ein Zeitpunkt der Zeit ermittelt, an welchem ein Signalintensitätsanteil der Signalintensität des jeweils nachzuweisenden Stoffes an einer Gesamtsignalintensität des MRT-Signals ein Maximum aufweist. Die Gesamtintensität des MRT-Signals setzt sich aus den Signalintensitäten der jeweiligen Stoffe zusammen. Es ist vorgesehen, dass der zumindest eine Zeitpunkt, an welchem der Signalintensitätsanteil der Signalintensität des jeweils nachzuweisenden Stoffes ein Maximum aufweist als Messzeitpunkt zum Erfassen des jeweiligen MRT-Signals zum Nachweis des jeweiligen Stoffes in dem Untersuchungsobjekt ermittelt wird. Mit anderen Worten ist es vorgesehen, dass der Zeitpunkt durch die Steuereinrichtung ermittelt wird zu welchem der durch den nachzuweisenden Stoff bereitgestellte Signalintensitätsanteil während der Magnetresonanzsequenz ein Maximum aufweist. Der ermittelte Zeitpunkt wird durch die Steuereinrichtung als Messzeitpunkt zum Nachweis des jeweils nachzuweisenden Stoffes in der Magnetresonanzsequenz festgelegt. Durch die Weiterbildung ergibt sich der Vorteil, dass zum Nachweis der Anwesenheit des jeweils nachzuweisenden Stoffes ein gezielt ermittelter Zeitpunkt ausgewählt wird.

Eine Weiterbildung der Erfindung sieht vor, dass durch die Steuereinrichtung eine Anweisung zum Nachweis von zumindest zwei der in dem Untersuchungsobjekt potentiell vorhandenen Stoffe empfangen wird. Mit anderen Worten ist es vorgesehen, dass die Steuereinrichtung eine Anweisung empfängt, welche die Steuereinrichtung dazu anleitet, zumindest zwei der potentiell vorhandenen Stoffe auf eine Anwesenheit in dem Untersuchungsobjekt zu überprüfen. Es kann beispielsweise vorgesehen sein, dass zumindest zwei der zumindest zwei in dem Untersuchungsobjekt vorhandenen Stoffe nachgewiesen werden sollen, oder mehr als zwei potentiell vorhandene Stoffe.

Es ist vorgesehen, dass für jeden der nachzuweisenden Stoffe zumindest ein jeweiliger Messzeitpunkt der Magnetresonanzsequenz durch die Steuereinrichtung ermittelt wird. Mit anderen Worten ist es vorgesehen, dass durch die Steuereinrichtung zum Nachweis der jeweiligen Stoffe jeweilige Messzeitpunkte ermittelt werden, zu welchen durch die Magnetresonanzvorrichtung die jeweiligen Signale erfasst werden, wobei ein jeweiliger Zeitpunkt zum Nachweis des jeweiligen Stoffes in dem Untersuchungsobjekt bestimmt ist. Es kann beispielsweise vorgesehen sein, dass durch die Magnetresonanzsequenz Signalverläufe für die jeweiligen nachzuweisenden Stoffe erzeugt werden. Zu einem ersten Zeitpunkt kann ein Signalintensitätsanteil eines ersten Stoffes ein Maximum aufweisen. Zu einem zweiten Zeitpunkt kann ein Signalintensitätsanteil eines zweiten Stoffes ein Maximum aufweisen. In diesem Fall kann der Zeitpunkt, zu welchem der Signalintensitätsanteil des ersten Stoffs das Maximum aufweist als Messzeitpunkt zum Erfassen des MRT-Signals zum Nachweis des ersten Stoffes bestimmt werden. Analog dazu kann der Zeitpunkt, zu welchem der Signalintensitätsanteil des zweiten Stoffs das Maximum aufweist durch die Steuereinrichtung als Messzeitpunkt zum Erfassen des MRT-Signals zum Nachweis des zweiten Stoffes durch die Steuereinrichtung bestimmt werden.

Eine Weiterbildung der Erfindung sieht vor, dass durch die Steuereinrichtung zum Nachweis der zumindest zwei in dem Untersuchungsobjekt nachzuweisenden Stoffe jeweilige Untersequenzen ermittelt werden. Mit anderen Worten ist es vorgesehen, dass die Magnetresonanzsequenz zumindest zwei der Untersequenzen aufweist, wobei die jeweiligen Untersequenzen zum Nachweis des jeweils nachzuweisenden Stoffes optimiert sind. Durch die Weiterbildung ergibt sich der Vorteil, dass zum Nachweis der jeweiligen Stoffe jeweils optimierte Untersequenzen bereitgestellt werden.

Eine Weiterbildung der Erfindung sieht vor, dass durch die Steuereinrichtung zumindest zwei Messzeitpunkte während der Magnetresonanzsequenz ermittelt werden und jeweilige Signalverläufe durch die Magnetresonanzvorrichtung erfasst werden. Mit anderen Worten ist es vorgesehen, dass anstatt einer Ermittlung einer Signalintensität eines MRT-Signals einem jeweiligen Messzeitpunkt ein Signalverlauf ermittelt wird. In einem weiteren Schritt ist es vorgesehen, dass der Verlauf der Signalintensität durch die Steuereinrichtung rekonstruiert wird. Die Steuereinrichtung vergleicht Verlauf der Signalintensität mit vorbestimmten Signalverläufen, wobei die vorbestimmten Signalverläufe jeweiligen Stoffen zugewiesen sind. Die Nachweisbedingung sieht dabei ein Erfüllen eines Übereinstimmungskriteriums des erfassten Signalverlaufs mit einem der vorbestimmten Signalverläufe des jeweiligen Stoffes vor. In Abhängigkeit von einem Maß einer Übereinstimmung des erfassten Signalverlaufs mit einem der vorbestimmten Signalverläufe wird die Anwesenheit des jeweiligen Stoffes bestätigt oder abgelehnt. Mit anderen Worten ist es vorgesehen, dass die Anwesenheit des zumindest einen nachzuweisenden Stoffes mittels einer Analyse des Signalintensitätsverlaufs überprüft wird. Es kann sich dabei beispielsweise um ein sogenanntes Fingerprinting-Verfahren zur Erfassung der Anwesenheit des jeweiligen Stoffes durch die Steuereinrichtung handeln. Im Gegensatz zum üblichen Fingerprinting-Verfahren wird jedoch eine auf den nachzuweisenden Stoff optimierte Magnetresonanzsequenz bereitgestellt.

Eine Weiterbildung der Erfindung sieht vor, dass die Magnetresonanzsequenz zumindest einen ortskodierten Sequenzabschnitt umfasst, und durch die Steuereinrichtung mittels des zumindest einen ortskodierten Sequenzabschnittes eine Lage des zumindest einen nachzuweisenden Stoffes in dem Untersuchungsobjekt ermittelt wird. Mit anderen Worten ist die Magnetresonanzsequenz derart beschaffen, dass diese eine Ortskodierung des MRT-Signals bewirkt. Mit anderen Worten ist die Magnetresonanzsequenz derart beschaffen, dass die Steuereinrichtung das jeweilige MRT-Signal einem jeweiligen Ort in dem Untersuchungsobjekt zuordnen kann. Die Magnetresonanzsequenz kann beispielsweise eine Gradientenechosequenz umfassen. Dadurch ergibt sich der Vorteil, dass zusätzlich zu der Anwesenheit des nachzuweisenden Stoffes in dem Untersuchungsobjekt dessen Lage in dem Untersuchungsobjekt ermittelt werden kann. Die Ortskodierung ist jedoch nicht zwingend erforderlich. Es kann vorgesehen sein, dass diese nur bei einem Nachweis des zumindest einen nachzuweisenden Stoffes erfolgt.

Eine Weiterbildung der Erfindung sieht vor, dass die Lage des zumindest einen nachzuweisenden Stoffes mit einer Bilddarstellung des Untersuchungsobjekts fusioniert wird. Es kann beispielsweise vorgesehen sein, dass die Magnetresonanzsequenz eine Bilderfassungssequenz zum Erfassen von Bilddaten umfasst. Die Steuereinrichtung kann aus den Bilddaten die Bilddarstellung des Untersuchungsobjektes rekonstruieren. Die Bilddarstellung kann auch durch die Steuereinrichtung empfangen werden. Die Lagen, an welchen der zumindest eine nachzuweisende Stoff erfasst ist, können in der Bilddarstellung farblich hervorgehoben werden. Es kann auch vorgesehen sein, dass in der Bilddarstellung vorhandene Strukturen durch die Steuereinrichtung als der zumindest eine nachzuweisende Stoff identifiziert werden können. In diesem Fall kann beispielsweise eine abgeschlossene Fläche einer ähnlichen Grauschattierung dem Stoff zugewiesen werden. Dadurch ergibt sich der Vorteil, dass Bilddarstellungen unter Berücksichtigung nachgewiesener Stoffe ausgewertet werden können. Eine Ortsauflösung bei dem Nachweis der Lage des Stoffes kann dabei auch geringer sein als eine Ortsauflösung der Bilddarstellung.

Eine Weiterbildung der Erfindung sieht vor, dass durch die Steuereinrichtung eine quantitative Größe für den zumindest einen nachzuweisenden Stoff ermittelt wird. Die quantitative Größe kann beispielsweise eine Wahrscheinlichkeit einer Anwesenheit des Stoffes in dem Untersuchungsobjekt oder zumindest dem einen Volumenelement, ein Anteil des Stoffes, oder eine absolute Menge des Stoffes betreffen. Dadurch ergibt sich der Vorteil, dass eine Quantifizierung des Stoffes ermöglicht werden kann. Die Quantifizierung kann sich auf das Objekt als Ganzes beziehen und/oder ortsabhängig sein. Bei einem Nachweis mehrerer Stoffe kann eine Zusammensetzung ermittelt werden. Die quantitative Größe kann in Bezug auf einen der Stoffe als Referenz bezogen sein.

Für Anwendungsfälle oder Anwendungssituationen, die sich bei dem Verfahren ergeben können und die hier nicht explizit beschrieben sind, kann vorgesehen sein, dass gemäß dem Verfahren eine Fehlermeldung und/oder eine Aufforderung zur Eingabe einer Nutzerrückmeldung ausgegeben und/oder eine Standardeinstellung und/oder ein vorbestimmter Initialzustand eingestellt wird.

Ein zweiter Aspekt der Erfindung betrifft eine Steuereinrichtung. Die Steuereinrichtung ist dazu eingerichtet, eine Magnetresonanzvorrichtung zum Nachweis eines vorgegebenen Stoffes in einem Untersuchungsobjekt anzusteuern. Die Steuereinrichtung ist dazu eingerichtet, eine Anweisung zum Nachweisen zumindest eines nachzuweisenden Stoffes zu empfangen. Um eine Anwesenheit des zumindest einen nachzuweisenden Stoffes in dem Untersuchungsobjekt zu ermöglichen, ist die Steuereinrichtung dazu eingerichtet, eine Magnetresonanzsequenz umfassend zumindest eine Untersequenz zum Nachweis des zumindest einen nachzuweisenden Stoffes in dem Untersuchungsobjekt zu ermitteln. Die Steuereinrichtung ist dazu eingerichtet, zumindest einen Messzeitpunkt zum Erfassen eines jeweiligen MRT-Signals zum Nachweisen des zumindest einen nachzuweisenden Stoffes in dem Untersuchungsobjekt zu ermitteln. Die Steuereinrichtung ist dazu eingerichtet, die Magnetresonanzvorrichtung zur Bereitstellung der ermittelten Magnetresonanzsequenz anzusteuern, und die Magnetresonanzvorrichtung zur Erfassung des MRT-Signals des Untersuchungsobjekts zu dem zumindest einen Messzeitpunkt anzusteuern. Die Steuereinrichtung ist dazu eingerichtet, das zumindest eine MRT-Signal zu empfangen, und eine Anwesenheit des zumindest einen nachzuweisenden Stoffes bei einer Erfüllung einer vorbestimmten Bedingung durch das zumindest eine MRT-Signal festzustellen.

Unter einer Steuereinrichtung kann insbesondere ein Datenverarbeitungsgerät verstanden werden, das einen Verarbeitungsschaltkreis enthält. Die Recheneinheit kann also insbesondere Daten zur Durchführung von Rechenoperationen verarbeiten. Darunter fallen gegebenenfalls auch Operationen, um indizierte Zugriffe auf eine Datenstruktur, beispielsweise eine Umsetzungstabelle, LUT (englisch: "look-up table"), durchzuführen.

Die Steuereinrichtung kann insbesondere einen oder mehrere Computer, einen oder mehrere Mikrocontroller und/oder einen oder mehrere integrierte Schaltkreise enthalten, beispielsweise eine oder mehrere anwendungsspezifische integrierte Schaltungen, ASIC (englisch: "application-specific integrated circuit"), eines oder mehrere feldprogrammierbare Gate-Arrays, FPGA, und/oder eines oder mehrere Einchipsysteme, SoC (englisch: "system on a chip"). Die Recheneinheit kann auch einen oder mehrere Prozessoren, beispielsweise einen oder mehrere Mikroprozessoren, eine oder mehrere zentrale Prozessoreinheiten, CPU (englisch: "central processing unit"), eine oder mehrere Grafikprozessoreinheiten, GPU (englisch: "graphics processing unit") und/oder einen oder mehrere Signalprozessoren, insbesondere einen oder mehrere digitale Signalprozessoren, DSP, enthalten. Die Recheneinheit kann auch einen physischen oder einen virtuellen Verbund von Computern oder sonstigen der genannten Einheiten beinhalten.

Ein dritter Aspekt der Erfindung betrifft eine Magnetresonanzvorrichtung, welche zumindest eine Steuereinrichtung aufweist.

Ein vierter Aspekt der Erfindung betrifft ein Computerprogrammprodukt. Weiterhin kann erfindungsgemäß ein Computerprogrammprodukt vorgesehen sein, welches direkt in einen Speicher einer Steuereinrichtung eines Magnetresonanztomographiesystems ladbar ist, mit Programmmitteln, um die Schritte des oben genannten Verfahrens auszuführen, wenn das Programm in der Steuereinrichtung des Magnetresonanztomographiesystems ausgeführt wird. Das hierin beschriebene Verfahren kann also auch in Form eines Computerprogrammprodukts vorliegen, das das Verfahren auf einer Steuereinheit implementiert, wenn es auf der Steuereinheit ausgeführt wird.

Ein fünfter Aspekt der Erfindung betrifft einen elektronisch lesbaren Datenträger.

Ebenso kann ein elektronisch lesbarer Datenträger mit darauf abgespeicherten elektronisch lesbaren Steuerinformationen vorliegen, welche zumindest ein beschriebenes Computerprogrammprodukt umfassen und derart ausgestaltet sind, dass sie bei der Verwendung des Datenträgers in einer Steuereinrichtung einer MR-Anlage ein beschriebenes Verfahren durchführen.
- FIG 1: zeigt eine schematische Darstellung einer beispielhaften Ausführungsform einer Magnetresonanzvorrichtung 1;
- FIG 2: zeigt eine schematische Darstellung eines Ablaufs einer beispielhaften Ausführungsform eines Verfahrens;
- FIG 3: zeigt eine schematische Darstellung einer Magnetresonanzsequenz; und
- FIG 4: zeigt eine schematische Darstellung einer Bildabbildung eines Untersuchungsobjektes mit lokalisierten Stoffen.

Die Magnetresonanzvorrichtung 1 weist eine Magneteinheit 10 mit einem Feldmagneten 11 auf, der ein statisches Magnetfeld zur Ausrichtung von Kernspins einer Probe, beispielsweise eines Patienten 100, in einem Untersuchungsbereich oder Aufnahmebereich erzeugt. Der Aufnahmebereich zeichnet sich durch ein äußerst homogenes statisches Magnetfeld auf, wobei die Homogenität insbesondere die Magnetfeldstärke beziehungsweise deren Betrag betrifft. Der Aufnahmebereich ist beispielweise nahezu kugelförmig und in einem Patiententunnel 16 positioniert, der sich in einer Längsrichtung 2 durch die Magneteinheit 10 erstreckt. Bei dem Feldmagneten 11 kann es sich beispielsweise um einen supraleitenden Magneten handelt, der magnetische Felder mit einer magnetischen Flussdichte von bis zu 3 T oder mehr bereitstellen kann. Für geringere Feldstärken können jedoch auch Permanentmagnete oder Elektromagnete mit normalleitenden Spulen Verwendung finden. Eine Patientenliege 30 kann in dem Patiententunnel 16 von einer Verfahreinheit 36 bewegbar sein.

Weiterhin weist die Magneteinheit 10 Gradientenspulen 12 auf, die dazu ausgelegt sind, zur räumlichen Differenzierung der erfassten Abbildungsbereiche in dem Aufnahmebereich dem statischen Magnetfeld ortsabhängige Magnetfelder in den drei Raumrichtungen zu überlagern. Die Gradientenspulen 12 können beispielsweise als Spulen aus normalleitenden Drähten ausgestaltet sein, die beispielsweise zueinander orthogonale Felder oder Feldgradienten in dem Aufnahmebereich erzeugen können.

Die Magneteinheit 10 kann als Sendeantenne beispielsweise eine Körperspule 14 aufweisen, die dazu ausgelegt ist, ein über eine Signalleitung zugeführtes Hochfrequenzsignal in den Untersuchungsbereich abzustrahlen. Die Körperspule 14 kann in manchen Ausführungsformen auch dazu genutzt werden, von dem Patienten 100 emittierte Resonanzsignale zu empfangen und über eine Signalleitung abzugeben. Die Körperspule 14 kann in solchen Ausführungsformen also als Hauptempfangsantenne sowie als Sendeantenne dienen. Daneben können aber auch spezielle für bestimmte Organe angepasste Spulen (sogenannte Lokalspulen) zum Empfang der Resonanzsignale verwendet werden.

Die Magnetresonanzvorrichtung 1 weist eine Steuereinheit 20 auf, die die Magneteinheit 10 mit verschiedenen Signalen für die Gradientenspulen 12 und die Körperspule 14 versorgen kann und die empfangenen Signale auswerten kann. Die Steuereinrichtung 20 kann beispielsweise eine Gradientensteuerung 21 aufweisen, die dazu ausgelegt ist, die Gradientenspulen 12 über Zuleitungen mit variablen Strömen zu versorgen, welche zeitlich koordiniert die gewünschten Gradientenfelder in dem Untersuchungsbereich bereitstellen können.

Die Steuereinrichtung 20 kann auch eine Hochfrequenzeinheit 22 aufweisen, die dazu ausgelegt ist, Hochfrequenzpulse oder Anregungspulse mit vorgegebenen zeitlichen Verläufen, Amplituden und spektraler Leistungsverteilung zur Anregung einer Magnetresonanz der Kernspins in dem Patienten 100 zu erzeugen. Dabei können Pulsleistungen im Bereich von Kilowatt eingesetzt werden. Die Anregungspulse können über die Körperspule 14 oder über eine oder mehrere lokale Sendeantennen in den Patienten 100 abgestrahlt werden. Die Steuereinrichtung 20 kann auch eine Steuerung 23 enthalten, die über einen Signalbus 25 mit der Gradientensteuerung 21 und der Hochfrequenzeinheit 22 kommunizieren kann.

Optional kann in der unmittelbaren Umgebung des Patienten 100, beispielsweise auf dem Patienten 100 oder in der Patientenliege 30, eine Lokalspule 50 angeordnet sein, die über eine Anschlussleitung 33 mit der Hochfrequenzeinheit 22 verbunden sein kann. Je nach Ausgestaltungsform kann die Lokalspule 50 alternativ oder zusätzlich zur Körperspule 14 als Hauptempfangsantenne dienen.

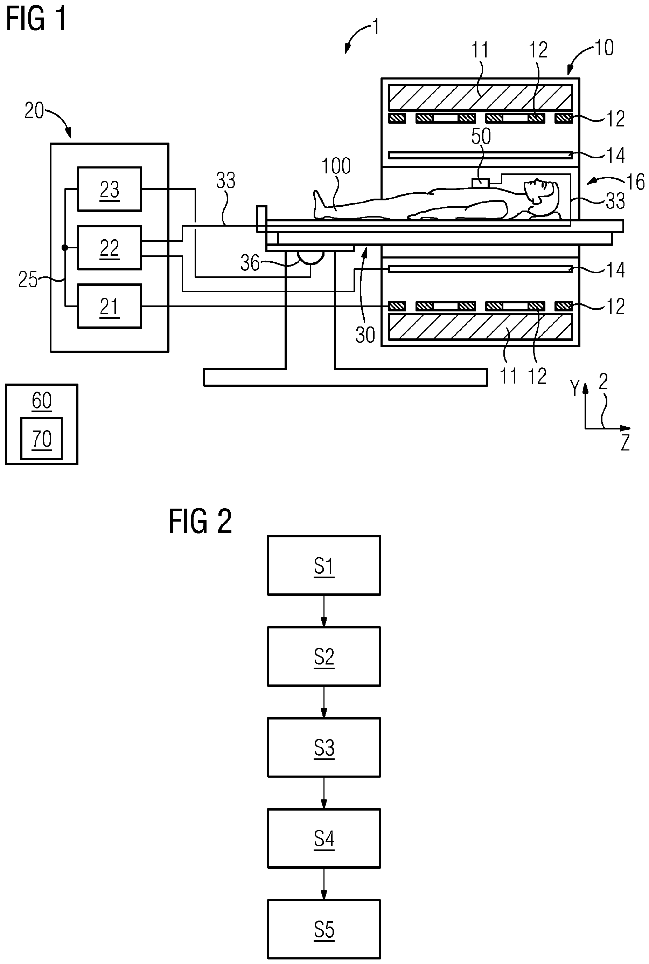

FIG 2 zeigt eine schematische Darstellung einer beispielhaften Ausführungsform eines Ablaufs eines Verfahrens zum Nachweis eines vorgegebenen Stoffes in einem Untersuchungsobjekt 100 durch eine Magnetresonanzvorrichtung 1 1.

In einem ersten Schritt S1 kann eine Information über zumindest einen nachzuweisenden Stoff, welcher potentiell in dem Untersuchungsobjekt 100 vorhanden ist, durch eine Steuereinrichtung 20 empfangen werden. Die Information kann auch zumindest einen potentiell vorhandenen weiteren Stoff umfassen. Die Information über den oder die potentiell vorhandenen Stoffe kann auch auf Vorwissen basieren und beispielsweise auf bekannten Gewebetypen im Gehirn/Kopf basieren. Das Vorwissen kann in diesem Fall in der Steuereinrichtung 20 gespeichert sein. Bei dem Untersuchungsobjekt 100 kann es sich um einen Körper aus anorganischem und/oder organischem Material handeln. Es kann sich bei dem Untersuchungsobjekt 100 um ein Lebewesen, insbesondere um einen Menschen handeln. Das Untersuchungsobjekt 100 kann mehrere Stoffe aufweisen. Es kann der Fall sein, dass die in dem Untersuchungsobjekt 100 vorhandenen Stoffe und/oder deren Lagen in dem Objekt unbekannt sein können. Um eine Anwesenheit zumindest eines der Stoffe nachweisen zu können, kann der Steuereinrichtung 20 die Information bereitgestellt werden, welche potentiell in dem Untersuchungsobjekt 100 vorhandene Stoffe potentiell vorhanden sind. Die Stoffe können durch Stoffparameter der jeweiligen Stoffe beschrieben sein, wie beispielsweise Relaxationsparameter T1, T2, T2* der jeweiligen Materialien. Die Steuereinrichtung 20 kann beispielsweise für die jeweiligen Stoffparameter ermitteln, welchen Verlauf eine Signalintensität B1, B2, B3 eines MRT-Signals A1, A2, A3 über eine Zeit aufweist, wenn der jeweilige Stoff mit einer Magnetresonanzsequenz MRT durch eine Magnetresonanzvorrichtung 1 angeregt wird. Der Verlauf kann beispielsweise nach einer Formel oder einer Simulation ermittelt werden.

In einem zweiten Schritt S2 kann durch die Steuereinrichtung 20 eine Anweisung zum Nachweisen zumindest eines der in dem Untersuchungsobjekt 100 potentiell vorhandenen Stoffe empfangen werden. Die Anweisung kann die Steuereinrichtung 20 dazu auffordern, die Magnetresonanzvorrichtung 1 zum Nachweis zumindest einer der Stoffe in dem Untersuchungsobjekt 100 anzusteuern. Die Steuereinrichtung 20 wird somit dazu angewiesen, die Magnetresonanzvorrichtung 1 derart anzusteuern, dass diese eine Magnetresonanzsequenz MRT zum Anregen des Untersuchungsobjektes 100 bereitstellt, um zumindest ein MRT-Signal A1, A2, A3 des Untersuchungsobjektes 100 zu erfassen, das zum Nachweis des zumindest einen nachzuweisenden Stoffes in dem Untersuchungsobjekt 100 geeignet ist.

In einem Schritt S3 kann durch die Steuereinrichtung 20 die Magnetresonanzsequenz MRT zum Nachweis des zumindest einen nachzuweisenden Stoffes nach einem Sequenzgenerierungsalgorithmus generiert werden. Die Magnetresonanzsequenz MRT kann eine oder mehrere Untersequenzen MRT1, MRT2 aufweisen, welche aufeinander folgen. Die Steuereinrichtung 20kann beispielsweise Sequenzparameter, wie Repetitionszeiten, die Inversionszeiten oder Flipwinkel der Untersequenzen MRT1, MRT2 ermitteln. Der Sequenzgenerierungsalgorithmus kann eine Formel, ein Modell oder eine Simulation zur Ermittlung der Magnetresonanzsequenz MRT vorsehen. Der Sequenzgenerierungsalgorithmus kann dazu vorgesehen sein, eine Magnetresonanzsequenz MRT dahingehend zu optimieren, dass der zumindest eine Stoff in dem Untersuchungsobjekt 100 durch das zumindest eine MRT-Signal A1, A2, A3 nachgewiesen werden kann. Die Ermittlung kann in Abhängigkeit von den zumindest zwei potentiell vorhandenen Stoffen erfolgen. Es können auch mehrere Untersequenzen MRT1, MRT2 durch den Sequenzgenerierungsalgorithmus ermittelt werden, um die jeweiligen nachzuweisenden Stoffe nachweisen zu können.

In einem Schritt S4 kann durch die Steuereinrichtung 20 zumindest ein Messzeitpunkt T1, T2, T3 zum Erfassen eines jeweiligen MRT-Signals A1, A2, A3 zum Nachweisen des zumindest einen nachzuweisenden Stoffes in dem Untersuchungsobjekt 100 in Abhängigkeit von dem zumindest einen nachzuweisenden Stoff alleine oder zusätzlich in Abhängigkeit von dem zumindest einen potentiell vorhandenen weiteren Stoff ermittelt wird. Mit anderen Worten wird durch die Steuereinrichtung 20 ein Messzeitpunkt T1, T2, T3 ermittelt, um ein geeignetes MRT-Signal A1, A2, A3 zum Nachweis des zumindest einen nachzuweisenden Stoffes erfassen zu können. Die Ermittlung des zumindest einen Messzeitpunktes T1, T2, T3 kann in Abhängigkeit jeweiliger Verläufe der Signalintensitäten B1, B2, B3 der jeweiligen Stoffe erfolgen. Die Verläufe der Signalintensitäten B1, B2, B3 der jeweiligen Stoffe können beispielsweise mittels Simulationen oder Formeln aus Stoffparametern der Stoffe ermittelt werden. Die Steuereinrichtung 20 kann beispielsweise den Messzeitpunkt T1, T2, T3 derart wählen, dass bei den Stoffparametern des zumindest einen nachzuweisenden Stoffes und den Stoffparametern des zumindest einen potentiell vorhandenen weiteren Stoffes davon ausgegangen werden kann, das ein zu dem Messzeitpunkt T1, T2, T3 erfasstes MRT-Signal A1, A2, A3 eine Gesamtintensität aufweist, die alleine oder mehrheitlich auf einer Signalintensität B1, B2, B3 des zumindest einen nachzuweisenden Stoffes beruht.

In einem Schritt S5 kann die Steuereinrichtung 20 die Magnetresonanzvorrichtung 1 ansteuern, damit durch diese die Magnetresonanzsequenz MRT bereitgestellt wird.

In einem Schritt S6 kann die Steuereinrichtung 20 die Magnetresonanzsequenz MRT ansteuern, damit diese das MRT-Signal A1, A2, A3 zu dem zumindest einen Messzeitpunkt T1, T2, T3 aufnimmt.

Informationen, wie eine Gesamtsignalintensität B des MRT-Signals A1, A2, A3, können durch die Magnetresonanzsequenz MRT an die Steuereinrichtung 20 versandt werden. In einem Schritt S7 können die Signaldaten durch die Steuereinrichtung 20 empfangen und ausgewertet werden. In der Auswertung kann zumindest überprüft werden, ob der zumindest eine nachzuweisende Stoff in dem Untersuchungsobjekt 100 vorhanden ist. Die Anwesenheit des zumindest einen nachzuweisenden Stoffes kann durch eine Überprüfung einer Erfüllung einer vorgegebenen Nachweisbedingung überprüft werden. Die Nachweisbedingung kann beispielsweise ein Überschreiten eines vorgegebenen Schwellenwertes durch die Gesamtintensität B des MRT-Signals A1, A2, A3 vorsehen. Ist die Nachweisbedingung erfüllt, kann durch die Steuereinrichtung 20 die Anwesenheit des zumindest einen nachzuweisenden Stoffes festgestellt werden und ein vorgegebenes Ausgabesignal ausgegeben werden. Falls die Nachweisbedingung nicht erfüllt wird, kann eine Ausgabe eines anderen Ausgabesignals vorgesehen sein.

Im Fall einer Erfassung des zumindest einen nachzuweisenden Stoffes können zusätzlich oder alternativ weitere durch die Steuereinrichtung 20 durchzuführende Schritte vorgesehen sein, wie beispielsweise eine Ermittlung einer quantitativen Größe in Bezug auf den zumindest einen nachzuweisenden Stoff, welche eine Menge, Dichte oder Anwesenheitswahrscheinlichkeit des Stoffes in dem Untersuchungsobjekt 100 oder zumindest einem Volumenelement angeben kann. Die quantitative Größe kann beispielsweise in Abhängigkeit einer Signalintensität B1 des nachzuweisenden Stoffes alleine oder im Vergleich zu einer Signalintensität B2eines weiteren der Stoffe ermittelt werden. Für die Ermittlung der quantitativen Größe kann bevorzugterweise das in Schritt S6 empfangene MRT-Signal A1 verwendet werden. Die Quantitative Größe kann beispielsweise aus der Signalintensität nach einem vorbestimmten Verfahren berechnet werden. Allerdings könnte hier eine hochaufgelöste Bildaufnahme getriggert werden.

Es kann auch eine Fusion mit Bilddaten des Objektes vorgesehen sein. In diesem Fall kann eine zusätzliche Sequenz zur Lokalisierung vorgesehen sein, falls dies in der Magnetresonanzsequenz MRT noch nicht erfolgt ist.

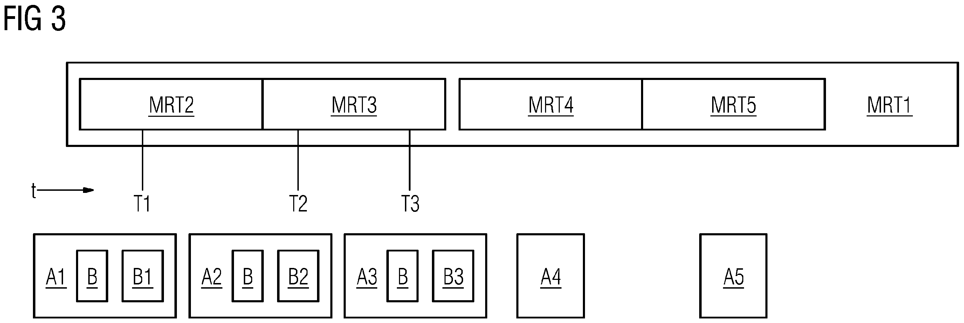

FIG 3 zeigt eine schematische Darstellung einer Magnetresonanzsequenz MRT.

Die Magnetresonanzsequenz MRT kann nach dem vorbestimmten Algorithmus generiert sein und zwei Untersequenzen MRT1, MRT2 aufweisen. Die Untersequenzen MRT1, MRT2 können dazu vorgesehen sein, einen Nachweis von Stoffen des Untersuchungsobjektes 100 zu ermöglichen. Eine erste Untersequenz kann zum Nachweis eines ersten der Stoffe vorgesehen sein. Zum Nachweis des ersten Stoffes kann ein erster Messzeitpunkt T1, T2, T3 vorgesehen sein, zu welchem ein erstes MRT-Signal A1, A2, A3 durch die Magnetresonanzvorrichtung 1 erfasst wird. Der erste Messzeitpunkt T1, T2, T3 kann derart gewählt sein, dass eine Gesamtsignalintensität B des ersten MRT-Signals A1, A2, A3 mit einer Signalintensität B1, B2, B3 des ersten Stoffes identisch ist. Dadurch kann es möglich sein, die Erfüllung der Nachweisbedingung durch die Signalintensität B1, B2, B3 des ersten Stoffes zu überprüfen.

Eine zweite Untersequenz kann zum Nachweis eines zweiten und eines dritten der Stoffe vorgesehen sein. Zum Nachweis des zweiten Stoffes kann ein zweiter Messzeitpunkt T1, T2, T3 vorgesehen sein, zu welchem ein zweites MRT-Signal A1, A2, A3 durch die Magnetresonanzvorrichtung 1 erfasst wird. Der zweite Messzeitpunkt T1, T2, T3 kann derart gewählt sein, dass eine Gesamtsignalintensität B des zweiten MRT-Signals A1, A2, A3 mit einer Signalintensität B1, B2, B3 des zweiten Stoffes identisch ist. Dadurch kann es möglich sein, die Erfüllung der Nachweisbedingung durch die Signalintensität B1, B2, B3 des zweiten Stoffes zu überprüfen.

Zum Nachweis des dritten Stoffes kann ein dritter Messzeitpunkt T1, T2, T3 vorgesehen sein, zu welchem ein drittes MRT-Signal A1, A2, A3 durch die Magnetresonanzvorrichtung 1 erfasst wird. Der dritte Messzeitpunkt T1, T2, T3 kann derart gewählt sein, dass eine Gesamtsignalintensität B des dritten MRT-Signals A1, A2, A3 mit einer Signalintensität B1, B2, B3 des dritten Stoffes identisch ist. Dadurch kann es möglich sein, die Erfüllung der Nachweisbedingung durch die Signalintensität B1, B2, B3 des dritten Stoffes zu überprüfen.

Es kann vorgesehen sein, dass bei einem Nachweis zumindest einer der Stoffe eine Lokalisierungssequenz MRT3 bereitgestellt wird, welche dazu vorgesehen sein kann, Lokalisierungssignale A4 bereitzustellen, welche eine Lokalisierung zumindest eines der Stoffe in dem Untersuchungsobjekt 100 ermöglicht. Die Lokalisierungssequenz MRT3 kann beispielsweise eine Gradientenechosequenz umfassen.

Es kann vorgesehen sein, dass die Magnetresonanzsequenz MRT eine Bilderfassungssequenz MRT4 umfasst, welche dazu vorgesehen sein kann, Bilderfassungssignale A5 bereitzustellen, welche eine Generierung einer Objektabbildung des Untersuchungsobjektes 100 durch die Steuereinrichtung 20 ermöglichen.

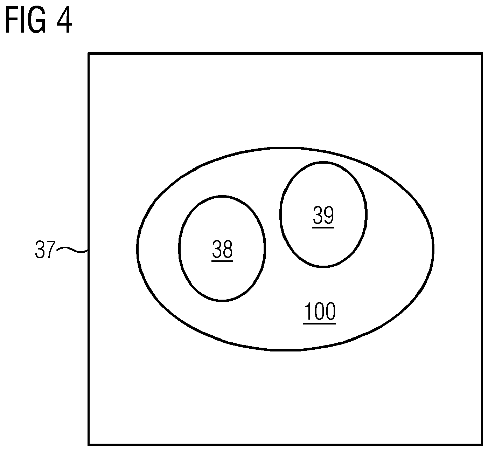

FIG 4 zeigt eine schematische Darstellung einer Objektabbildung 37 eines Untersuchungsobjektes 100 mit lokalisierten Stoffen.

Die Objektabbildung 37 kann eine zweidimensionale oder dreidimensionale Abbildung des Untersuchungsobjektes 100 sein. In der Objektabbildung 37 können Stoffverteilungen 38, 39 jeweiliger nachzuweisender Stoffe angezeigt sein.

Die Erfindung beruht auf dem Gedanken, Magnetresonanzsequenzen MRT von HF-Impulsen und Gradientenimpulsen zu ermitteln, die zu bestimmten Abtastzeitpunkten zu Signalen führen, welche nur einem spezifischen Stoff und/oder Gewebe zuzuordnen sind. Dadurch kann ein Untersuchungsobjekt 100 beispielsweise auf ein Vorhandensein eines nachzuweisenden Stoffes, beispielsweise eines kanzerogenen Gewebes oder Läsionen ohne Bildgebung bestätigt oder verneint werden. Das Verfahren könnte auch für die Erkennung und/oder Bestimmung von bestimmtem Gewebe oder gutartigen oder bösartigen Veränderungen verwendet werden.

Im Gegensatz zu den allgemeinen MRT-Fingerprinting-Verfahren ist es vorgesehen, eine Magnetresonanzsequenz MRT zu ermitteln, welche individuell zur Erfassung des nachzuweisenden Stoffes generiert sein kann. Dadurch kann es möglich sein, die Magnetresonanzsequenz MRT zur Durchführung eines Schnell-screening-Verfahrens zu verwenden. Die Magnetresonanzsequenz MRT kann nach einem vorbestimmten Algorithmus generiert sein, und darauf optimiert sein, dass die Magnetresonanzsequenz MRT eine HF-Sequenz und/oder eine Gradientensequenz der Magnetresonanzsequenz MRT so gestaltet, dass das MRT-Signal A1, A2, A3 eine Gesamtsignalintensität B aufweist, welche lediglich eine Signalintensität B1, B2, B3 umfasst, die auf den nachzuweisenden Stoff zurückzuführen ist.

Dies kann dadurch erreicht werden, indem die Magnetresonanzsequenz MRT basierend auf stoffspezifischen Parameterkombinationen generiert wird. Die stoffspezifischen Parameter können die Relaxationswerte T1 und T2, T2* und/oder zusätzliche Parameter wie den scheinbaren Diffusions-Koeffizienten, kurz ADC, umfassen. Die HF-Sequenz und/oder die Gradientensequenz der Magnetresonanzsequenz MRT kann durch den Algorithmus so optimiert werden, dass eine Signalintensität B1, B2, B3 des nachzuweisenden Stoffes für diese Parameterkombinationen maximiert wird. Die Magnetresonanzsequenz MRT kann durch den Algorithmus auch dahingehend optimiert werden, dass die Signalintensität B1, B2, B3 des nachzuweisenden Stoffes maximiert und die Signalintensität B1, B2, B3 weiterer möglicher Stoffe des Untersuchungsobjektes 100 minimiert wird. Dadurch kann es möglich sein, einen Signalintensitätsanteil der Signalintensität B1, B2, B3 des nachzuweisenden Stoffes an einer Gesamtsignalintensität B, welche die Signalintensität B1, B2, B3 aller Stoffe des Untersuchungsobjekts 100 umfassen kann, zu maximieren. Der Algorithmus kann auf einer Funktion, einem Modell, einer Tabelle und/oder einer Simulation basieren.

Das Verfahren kann darauf gerichtet sein, lediglich eine Anwesenheit des nachzuweisenden Stoffes in dem Untersuchungsobjekt 100 festzustellen. In diesem Fall kann es vorgesehen sein, das Verfahren derart durchzuführen, dass keine oder nur eine relativ grobe Lokalisierung des nachzuweisenden Stoffes erfolgt. Die Magnetresonanzsequenz MRT kann in diesem Fall beispielsweise zur gezielten Anregung der Prostataregion ausgelegt sein. Anhand einer Erfassung eines MRT-Signals A1, A2, A3, dessen Signalintensität B1, B2, B3 auf den nachzuweisenden Stoff zurückzuführen ist, könnte dann direkt die Frage beantworten, ob und wie viel von dem nachzuweisenden Stoff in der Zielregion des Untersuchungsobjektes 100 vorhanden ist. Das Vorgehen wäre in diesem Fall vergleichbar mit einem Single-Voxel-Spektroskopie-Ansatz, bei dem das Vorhandensein bestimmter Metaboliten in einem großen Voxel nachgewiesen wird. Es kann vorgesehen sein, dass in dem Fall, dass keine relevante Signalintensität B1, B2, B3 vorliegt, davon ausgegangen wird, dass der nachzuweisende Stoff nicht vorhanden ist. Dann kann ein entsprechendes Ausgabesignal durch die Steuereinrichtung 20 ausgegeben werden. Für diesen Fall sind keine weiteren Messungen mehr nötig.

Wird eine vorbestimmte Nachweisbedingung erfüllt, beispielsweise ein vorbestimmter Schwellenwert durch die Signalintensität B1, B2, B3 überschritte, kann die Anwesenheit des nachzuweisenden Stoffes festgestellt werden. In Abhängigkeit von dem Signalintensitätswert kann durch die Steuereinrichtung 20 ein quantitativer Wert ermittelt und ausgegeben werden, welcher beispielsweise eine Wahrscheinlichkeit des Vorliegens des nachzuweisenden Stoffes, einen Anteil des Stoffes oder eine Menge des nachzuweisenden Stoffes in dem Untersuchungsobjekt 100 oder dem Bereich beschreiben kann.

In diesem Fall kann es vorgesehen sein, dass eine genauere Lageermittlung zur Ermittlung der Lage des zumindest einen nachzuweisenden Stoffes durchgeführt wird. Die Steuereinrichtung 20 kann hierfür die Magnetresonanzsequenz MRT um einen Sequenzabschnitt ergänzen, welcher eine Lokalisierung des zumindest einen nachzuweisenden Stoffes in dem Untersuchungsobjekt 100 ermöglicht und die Magnetresonanzvorrichtung 1 zur Bereitstellung der zusätzlichen Sequenz ansteuern. Dies kann beispielsweise über eine Gradientenechosequenz ermöglicht werden. Dabei erfasste Signale können an die Steuereinrichtung 20 übertragen werden, welche daraus die Lage des zumindest einen nachzuweisenden Stoffes ermitteln kann.

Es kann zusätzlich vorgesehen sein, dass Daten über die ermittelte Lage mit Bildaufnahmen des Untersuchungsobjektes 100 fusioniert werden. Die Bildaufnahmen können der Steuereinrichtung 20 bereitgestellt werden. Es kann auch vorgesehen sein, dass die Magnetresonanzvorrichtung 1 durch die Steuereinrichtung 20 zur Durchführung eines bildgebenden Scans des Untersuchungsobjekts 100 zur Erfassung einer 2D- oder 3D-Bildanalyse angesteuert wird. Die Bildanalyse kann durch die Steuereinrichtung 20 erfolgen. Die Anwesenheit oder eine ermittelte Lage kann mit der Bildanalyse fusioniert werden.

Eine andere Teilimplementierung könnte eine weitere Signallokalisierung nutzen, die optional mit lokalen Spulenelementen oder konventioneller Gradientencodierung in einer Gradientenechosequenz der Magnetresonanzsequenz MRT durchgeführt werden kann, was zu einer lokalisierten Bildgebungstechnik führt, bei der auch die individuelle Lage des zumindest einen nachzuweisenden Stoffes in dem Untersuchungsobjekt 100 ermittelt wird. Die Magnetresonanzsequenz MRT könnte von Anfang an eine Gradienten- oder Spinechosequenz zur Lageermittlung umfassen. Dies ist vergleichbar mit der Bildgebung in der Positronen-Emissions-Tomographie, kurz PET, mit einem spezifischen Tracer. Für den anatomischen Kontext kann dieses Bild auf hochauflösende MR-Bildern des Untersuchungsobjektes 100 überlagert werden.

Ein weiteres Merkmal könnte sein, dass die Magnetresonanzsequenz MRT nicht nur zum Nachweis des zumindest einen nachzuweisenden Stoffes in dem Untersuchungsobjekt 100 kodiert sein kann, sondern für zwei, drei oder mehr nachzuweisende Stoffe. Die Anwesenheit der nachzuweisenden Stoffe kann während der Magnetresonanzsequenz MRT überprüft werden. Zum Nachweis der unterschiedlichen Stoffe kann ein MRT-Signal A1, A2, A3 an einem gemeinsamen Messpunkt oder an unterschiedlichen Messpunkten vorgesehen sein. Es kann auch vorgesehen sein, dass für einen jeweiligen Stoff eine jeweilige Untersequenz MRT1, MRT2 ermittelt wird. Es kann auch vorgesehen sein, dass eine Untersequenz MRT1, MRT2 für zumindest zwei der Stoffe vorgesehen sein kann. Die Untersequenzen MRT1, MRT2 können zeitlich aufeinander folgen. Es kann vorgesehen sein, dass die nachzuweisenden Stoffe in unterschiedlichen Farbkodierungen mit der Bildaufnahme fusioniert, beispielsweise kombiniert oder überlagert werden.

Dasselbe kann in einer einzigen Aufnahme geschehen, in der die jeweiligen Parameterkombinationen farblich kodiert sind.

Der Algorithmus kann auch dazu eingerichtet sein, dass er anstatt eine Magnetresonanzsequenz MRT zu generieren, die nur zum Nachweis eines einzigen Stoffes oder einer Gruppe von Stoffen geeignet ist, weil nur diese einen Signalintensitätsanteil an einer Gesamtintensität eines MRT-Signal A1, A2, A3 liefern, die Magnetresonanzsequenz MRT so generieren, dass zu verschiedenen Messzeitpunkten T1, T2, T3 der Magnetresonanzsequenz MRT jeweilige Signale erfasst werden können, deren Signalintensitäten B1, B2, B3 ein jeweiliger Stoff zugeordnet werden kann.

Bei mehreren Geweben, deren Anwesenheit entweder kombiniert oder nacheinander überprüft wird, könnte einer dieser nachzuweisenden Stoffe als Referenzstoff dienen. Dies könnte eine bessere Quantifizierung ermöglichen. Es könnte vorgesehen sein, dass in einem Verfahren eine Magnetresonanzsequenz MRT bereitgestellt wird, für die zwei Messzeitpunkte T1, T2 ermittelt werden. Ein erster der Messzeitpunkte T1 kann derart gewählt sein, dass eine Gesamtsignalintensität B des zu dem Messzeitpunkt T1 erfassten MRT-Signals A1 alleine auf die Signalintensität B1, zum Beispiel von Fett als nachzuweisenden Stoff, zurückzuführen ist. Mit anderen Worten ist dieser Messzeitpunkt T1 so gewählt, dass nur Fett in dem MRT-Signal A1 erfasst wird. Ein weiterer Messzeitpunkt T2 kann so gelegt sein, dass nur die Signalintensität B2, zum Beispiel von Wasser, in der Gesamtsignalintensität B des MRT-Signal A2 erfasst wird. Die Intensität des MRT-Signals A1, A2, A3 einer der Stoffe kann als quantitative Referenz verwendet werden. Durch diese Referenz kann die quantitative Größe eines weiteren Stoffes in Bezug zu ihrer Signalintensität B1, B2, B3 gesetzt werden.

Das Verfahren kann statt der Erkennung einer Anwesenheit eines Stoffes anhand von dessen Signalintensität B1, B2, B3 an zumindest einem Messpunkt eine Anwesenheit des Stoffes anhand eines Signalverlaufs der Signalintensität B1, B2, B3 vorsehen. Die Magnetresonanzsequenz MRT kann derart gestaltet sein, dass ein Verlauf einer Signalintensität B1, B2, B3 eines nachzuweisenden Stoffes gut von den Verläufen der Signalintensitäten B1, B2, B3 anderer Stoffe unterscheidbar ist. Mit anderen Worten umfasst der Verlauf der Gesamtintensität die Verläufe der Intensitäten der Stoffe. Anstatt die Magnetresonanzsequenz MRT so zu gestalten, dass an zumindest einem Messzeitpunkt T1, T2, T3 nur die Signalintensität B1, B2, B3 eines der nachzuweisenden Stoffe erfasst wird und die Signalintensitäten B1, B2, B3 anderer Stoffe minimiert sind, ist es in diesem Fall nicht erforderlich, bestimmte Signalanteile zu unterdrücken. Die einzelnen Verläufe müssen nur derart verlaufen, dass sich diese in dem Verlauf der Gesamtintensität erfassen lassen. Ein Algorithmus kann zum Erfassen der Verläufe vorgesehen sein. Der Algorithmus kann beispielsweise einen einfachen Musterabgleich oder eine Identifikation eines Verlaufs mittels eines neuronalen Netzes vorsehen. Der Algorithmus ist in der Lage zu erkennen, ob der Verlauf der Signalintensität B1, B2, B3 des Stoffes in dem erfassten Verlauf der Gesamtsignalintensität B enthalten ist. Eine Generierung einer Magnetresonanzsequenz MRT, die zur Lokalisierung geeignet ist, ist in diesem Fall nicht erforderlich. Dieser Ansatz kann ohne Signallokalisierung durchgeführt werden, um eine Schätzung zu erhalten, ob der zumindest eine Stoff in dem gescannten Volumen des Untersuchungsobjekts 100 enthalten ist.

Der Ansatz kann aber auch mit Lokalisierungstechniken, wie der Gradientenkodierung durch die Magnetresonanzsequenz MRT kombiniert werden, um eine Abbildung mit einer Kartierung des nachzuweisenden Stoffes erzeugen zu können, wie es bereits erwähnt wurde.

Das Ergebnis des Erkennungsschritts kann eine quantitative Größe, beispielsweise eine Mengeninformation oder eine Wahrscheinlichkeit des nachzuweisenden Stoffes umfassen.

Die Sequenzoptimierung von HF- und Gradientenimpulsen kann entweder analytisch, numerisch oder mit Hilfe von Simulationen durchgeführt werden. Zur besseren Signaltrennung kann die Magnetresonanzsequenz MRT weitere Parameter, wie zum Beispiel Diffusionsgewichtungen und/oder Magnetisierungstransfers berücksichtigen.

Diese Schnell-Screening-Scans können Teil von automatisierten Arbeitsabläufen sein. Wenn ein verdächtiges MRT-Signal A1, A2, A3 gefunden wurde, kann es durch das beschriebene Verfahren klassifiziert werden, indem eine Magnetresonanzsequenz MRT zur Erfassung eines nachzuweisenden Stoffes generiert und durch die Magnetresonanzvorrichtung 1 bereitgestellt wird.

Je nach Ergebnis können zeitaufwändigere Scans gewählt werden, die auf die vermutete Pathologie zugeschnitten sind.

Das schnelle Screening könnte auch zusätzlich zu speziellen lokalen Messungen eingesetzt werden. Da sich das Untersuchungsobjekt 100 bereits in der Magnetresonanzvorrichtung 1 befindet, könnte das Screening im Grunde zu jeder Standard-MR-Untersuchung hinzugefügt werden.

Im Gegensatz zu früheren Techniken, einschließlich MR-Fingerprinting, wäre in dem beschriebenen Verfahren keine Bildaufnahme und -analyse erforderlich. Anhand des erfassten MRT-Signals A1, A2, A3 könnte eine Anwesenheit und eine Menge des Stoffes in einem Volumenelement des Untersuchungsobjektes 100 ermittelt werden. Dies könnte die Erfassung und Datenanalyse erheblich beschleunigen.

## Patentansprüche

1. Verfahren zum Nachweis eines vorgegebenen nachzuweisenden Stoffes in einem Untersuchungsobjekt (100) durch eine Magnetresonanzvorrichtung (1),
wobei durch eine Steuereinrichtung (20)
- eine Anweisung zum Nachweisen zumindest eines in dem Untersuchungsobjekt (100) nachzuweisenden Stoffes empfangen wird,
- eine Magnetresonanzsequenz (MRT) umfassend zumindest eine Untersequenz (MRT1, MRT2) zum Nachweis des zumindest einen nachzuweisenden Stoffes in dem Untersuchungsobjekt (100) in Abhängigkeit von dem zumindest einen nachzuweisenden Stoff ermittelt wird,
- zumindest ein Messzeitpunkt (T1, T2, T3) in der Magnetresonanzsequenz zum Erfassen eines jeweiligen MRT-Signals (A1, A2, A3) zum Nachweisen des zumindest einen nachzuweisenden Stoffes in dem Untersuchungsobjekt (100) in Abhängigkeit von dem zumindest einen nachzuweisenden Stoff ermittelt wird,
- eine Magnetresonanzvorrichtung (1) zur Bereitstellung der ermittelten Magnetresonanzsequenz (MRT) angesteuert wird,
- die Magnetresonanzvorrichtung (1) zur Erfassung des zumindest einen MRT-Signals (A1, A2, A3) des Untersuchungsobjekts (100) zu dem zumindest einen Messzeitpunkt (T1, T2, T3) in der Magnetresonanzsequenz (MRT) angesteuert wird,
- das zumindest eine MRT-Signal (A1, A2, A3) auf eine Erfüllung einer vorbestimmten Nachweisbedingung ausgewertet wird, und bei einer Erfüllung der vorbestimmten Nachweisbedingung durch das zumindest eine MRT-Signal (A1, A2, A3) eine Anwesenheit des zumindest einen nachzuweisenden Stoffes festgestellt wird.

2. Verfahren nach Anspruch 1,
wobei durch die Steuereinrichtung (20)
- eine Information über zumindest einen weiteren Stoff, welcher potentiell in dem Untersuchungsobjekt (100) vorhanden ist empfangen wird,
- die Magnetresonanzsequenz (MRT) in Abhängigkeit von dem zumindest einen weiteren potentiell vorhandenen Stoff ermittelt wird,
- der zumindest eine Messzeitpunkt (T1, T2, T3) zum Erfassen des jeweiligen MRT-Signals (A1, A2, A3) zum Nachweisen des zumindest einen nachzuweisenden Stoffes in dem Untersuchungsobjekt (100) in Abhängigkeit von dem zumindest einen potentiell vorhandenen weiteren Stoff ermittelt wird.

3. Verfahren nach Anspruch 2, wobei die Untersequenz (MRT1, MRT2) zum Nachweis des zumindest einen nachzuweisenden Stoffes in dem Untersuchungsobjekt (100) nach einem vorbestimmten Sequenzgenerierungsalgorithmus in Abhängigkeit von Stoffparametern des zumindest einen nachzuweisenden Stoffes und/oder Stoffparametern des zumindest einen potentiell vorhandenen weiteren Stoffes ermittelt wird, wobei der vorbestimmte Sequenzgenerierungsalgorithmus dazu eingerichtet ist, die Untersequenz (MRT1, MRT2) gemäß eines vorgegebenen Optimierungskriteriums zu generieren.

4. Verfahren nach Anspruch 3, wobei das vorbestimmte Optimierungskriterium eine Maximierung einer Signalintensität (B1, B2, B3) des MRT-Signals (A1, A2, A3) für den zumindest einen nachzuweisenden Stoff an zumindest einem Zeitpunkt der Magnetresonanzsequenz (MRT) vorgibt.

5. Verfahren nach Anspruch 3 oder 4, wobei das vorbestimmte Optimierungskriterium eine Minimierung einer Signalintensität (B1, B2, B3) des MRT-Signals (A1, A2, A3) für den zumindest einen potentiell vorhandenen weiteren Stoff an zumindest einem Zeitpunkt der Magnetresonanzsequenz (MRT) vorgibt.

6. Verfahren nach einem der Ansprüche 3 bis 5, wobei das vorbestimmte Optimierungskriterium eine Maximierung eines Signalanteils der Signalintensität (B1, B2, B3) an einer Gesamtsignalintensität (B1, B2, B3) (B) des MRT-Signals (A1, A2, A3) für den zumindest einen nachzuweisenden Stoff an zumindest einem Zeitpunkt der Magnetresonanzsequenz (MRT) vorgibt.

7. Verfahren nach einem der Ansprüche 3 bis 6, wobei das vorbestimmte Optimierungskriterium eine Minimierung eines Signalanteils der Signalintensität (B1, B2, B3) an einer Gesamtsignalintensität (B1, B2, B3) (B) des MRT-Signals (A1, A2, A3) für den zumindest einen potentiell vorhandenen weiteren Stoff an zumindest einem Zeitpunkt der Magnetresonanzsequenz (MRT) vorgibt.

8. Verfahren nach einem der vorhergehenden Ansprüche, wobei durch die Steuereinrichtung (20) für den zumindest einen nachzuweisenden Stoff
- zumindest zwei mögliche Untersequenzen (MRT1, MRT2) zum Nachweisen des jeweiligen Stoffes ermittelt werden,
- für die zumindest zwei möglichen Untersequenzen (MRT1, MRT2) jeweilige Verläufe jeweiliger Signalintensitäten (B1, B2, B3) des MRT-Signals (A1, A2, A3) für die jeweiligen Stoffe über eine Zeit der möglichen Untersequenzen (MRT1, MRT2) ermittelt werden,
- eine der zumindest zwei möglichen Untersequenzen (MRT1, MRT2) in Abhängigkeit von zumindest einem der Signalverläufe nach einem vorbestimmten Auswahlkriterium als Untersequenz (MRT1, MRT2) zum Nachweis des zumindest einen nachzuweisenden Stoffes ausgewählt wird.

9. Verfahren nach Anspruch 8 in dessen Rückbezug auf Anspruch 2,
wobei durch die Steuereinrichtung (20)
- für die jeweiligen möglichen Untersequenzen (MRT1, MRT2) ein jeweiliger Maximalwert eines Signalintensitätsanteils der Signalintensität (B1, B2, B3) des MRT-Signals (A1, A2, A3) für den zumindest einen nachzuweisenden Stoff an einer Gesamtsignalintensität (B) des MRT-Signals (A1, A2, A3) ermittelt wird, wobei die Gesamtintensität des MRT-Signals (A1, A2, A3) sich aus den Signalintensitäten (B1, B2, B3) der jeweiligen Stoffe zusammensetzt, und
- das Auswahlkriterium ein Aufweisen eines größten Maximalwerts des Signalintensitätsanteils des nachzuweisenden Stoffes aufweist.

10. Verfahren nach einem der vorhergehenden Ansprüche, wobei durch die Steuereinrichtung (20)
- für die Magnetresonanzsequenz (MRT) jeweilige Verläufe jeweiliger Signalintensitäten (B1, B2, B3) des MRT-Signals (A1, A2, A3) für die jeweiligen Stoffe über eine Zeit der ermittelten Magnetresonanzsequenz (MRT) ermittelt werden,
- zumindest ein Zeitpunkt der Zeit ermittelt wird, an welchem ein Signalintensitätsanteil der Signalintensität (B1, B2, B3) des jeweiligen nachzuweisenden Stoffes an einer Gesamtsignalintensität (B) des MRT-Signals (A1, A2, A3) ein Maximum aufweist, wobei die Gesamtintensität des MRT-Signals (A1, A2, A3) sich aus den Signalintensitäten (B1, B2, B3) der jeweiligen Stoffe zusammensetzt, und
- der zumindest eine Zeitpunkt, an welchem der Signalintensitätsanteil der Signalintensität (B1, B2, B3) des jeweiligen nachzuweisenden Stoffes das Maximum aufweist, als Messzeitpunkt (T1, T2, T3) zum Erfassen des jeweiligen MRT-Signals (A1, A2, A3) zum Nachweisen des jeweiligen Stoffes in dem Untersuchungsobjekt (100) ermittelt wird.

11. Verfahren nach einem der vorhergehenden Ansprüche, wobei durch die Steuereinrichtung (20) eine Anweisung zum Nachweisen von zumindest zwei in dem Untersuchungsobjekt (100) nachzuweisenden Stoffe empfangen wird, und für jeden der nachzuweisenden Stoffe zumindest ein jeweiliger Messzeitpunkt (T1, T2, T3) zum Erfassen des jeweiligen MRT-Signals (A1, A2, A3) zum Nachweisen des jeweiligen nachzuweisenden Stoffes in dem Untersuchungsobjekt (100) ermittelt wird.

12. Verfahren nach Anspruch 11, wobei durch die Steuereinrichtung (20) für jeden der nachzuweisenden Stoffe zumindest eine jeweilige Untersequenz (MRT1, MRT2) zum Erfassen des jeweiligen MRT-Signals (A1, A2, A3) zum Nachweisen des jeweiligen nachzuweisenden Stoffes in dem Untersuchungsobjekt (100) ermittelt wird.

13. Verfahren nach einem der vorhergehenden Ansprüche, wobei durch die Steuereinrichtung (20)
- an zumindest zwei Messzeitpunkten (T1, T2, T3) während der Magnetresonanzsequenz (MRT) jeweilige Gesamtsignalintensitäten (B) der MRT-Signale (A1, A2, A3) erfasst werden,
- ein Verlauf der Gesamtsignalintensität (B) aus den Gesamtintensitäten (B) der zumindest zwei Messzeitpunkte (T1, T2, T3) ermittelt wird,
- der Verlauf der Gesamtsignalintensität (B) mit zumindest einem vorbestimmten Signalverlauf verglichen wird, wobei der vorbestimmte Signalverlauf dem zumindest einem nachzuweisendem Stoff zugewiesen ist,
wobei
die Nachweisbedingung zum Nachweis des zumindest einen nachzuweisenden Stoffes ein Erfüllen eines Übereinstimmungskriteriums des erfassten Verlaufs der Gesamtsignalintensität (B) in Bezug auf den vorbestimmten Signalverlauf des jeweiligen nachzuweisenden Stoffes umfasst.

14. Verfahren nach einem der vorhergehenden Ansprüche, wobei die Magnetresonanzsequenz (MRT) zumindest einen ortskodierten Sequenzabschnitt umfasst, und durch die Steuereinrichtung (20) mittels des zumindest einen ortskodierten Sequenzabschnittes eine Lage des zumindest einen nachzuweisenden Stoffes in dem Untersuchungsobjekt (100) ermittelt wird.

15. Verfahren nach Anspruch 14, wobei durch die Steuereinrichtung (20) die Lage des zumindest einen nachzuweisenden Stoffes mit einer Bilddarstellung des Untersuchungsobjekts (100) fusioniert wird.

16. Verfahren nach einem der vorhergehenden Ansprüche,
wobei durch die Steuereinrichtung (20)
eine quantitative Größe für den zumindest einen nachzuweisenden Stoff ermittelt wird.

17. Steuereinrichtung (20), eingerichtet zur Steuerung (23) einer Magnetresonanzvorrichtung (1) zum Nachweis eines vorgegebenen nachzuweisenden Stoffes in einem Untersuchungsobjekt (100),
wobei die Steuereinrichtung (20) dazu eingerichtet ist,
- eine Anweisung zum Nachweisen zumindest eines in dem Untersuchungsobjekt (100) nachzuweisenden Stoffes zu empfangen,
- eine Magnetresonanzsequenz (MRT) umfassend zumindest eine Untersequenz (MRT1, MRT2) zum Nachweis des zumindest einen nachzuweisenden Stoffes in dem Untersuchungsobjekt (100) in Abhängigkeit von dem zumindest einen nachzuweisenden Stoff zu ermittelt,
- zumindest einen Messzeitpunkt (T1, T2, T3) in der Magnetresonanzsequenz zum Erfassen eines jeweiligen MRT-Signals (A1, A2, A3) in Abhängigkeit von dem zumindest einen nachzuweisenden Stoff zum Nachweisen des zumindest einen nachzuweisenden Stoffes in dem Untersuchungsobjekt (100) zu ermitteln,
- die Magnetresonanzvorrichtung (1) zur Bereitstellung der ermittelten Magnetresonanzsequenz (MRT) anzusteuern,
- die Magnetresonanzvorrichtung (1) zur Erfassung des zumindest einen MRT-Signals (A1, A2, A3) des Untersuchungsobjekts (100) zu dem zumindest einen Messzeitpunkt (T1, T2, T3) anzusteuern,
- das zumindest eine MRT-Signal (A1, A2, A3) zu empfangen, und eine Anwesenheit des zumindest einen nachzuweisenden Stoffes bei einer Erfüllung einer vorbestimmten Bedingung durch das zumindest eine MRT-Signal (A1, A2, A3) festzustellen.

18. Magnetresonanzvorrichtung (1), aufweisend zumindest eine Steuereinrichtung (20) nach Anspruch 17.

19. Computerprogramm (70), welches direkt in einen Speicher einer Steuereinrichtung (20) nach Anspruch 14 ladbar ist, mit Programm-Mitteln, um die Schritte des Verfahrens nach einem der Ansprüche 1 bis 16 auszuführen, wenn das Programm in der Steuereinrichtung (20) ausgeführt wird.

20. Elektronisch lesbarer Datenträger mit darauf gespeicherten elektronisch lesbaren Steuerinformationen, welche zumindest ein Computerprogramm nach Anspruch 16 umfassen und derart ausgestaltet sind, dass sie bei Verwendung des Datenträgers in einer Steuereinrichtung (2) nach Anspruch 17 ein Verfahren nach einem der Ansprüche 1 bis 16 durchführen.
